# EUROPEAN PATENT APPLICATION

(11) **EP 1 504 793 A2**
(43) Date of publication of application: **09.02.2005**
(21) Application number: 04103622.9
(22) Date of filing: 28.07.2004
(51) Int. Cl.: A61N 5/10, G03C 5/16

(54) **X-ray imaging cassette for radiotherapy**

(30) Priority: 30.07.2003 EP 31023419
(71) Applicant: AGFA-GEVAERT, 2640 Mortsel (BE)
(72) Inventor: Koninckx, Jan, 2640, Mortsel (BE)

(57) **Abstract**

An X-ray imaging cassette having a cover side and a tube side comprises, inbetween said cover and tube side, a radiation image storage phosphor plate and a tungsten filter foil having a thickness in the range from 0.10 to 0.60 mm, and, more preferably in the range from 0.10 to 0.30 mm, and is particularly useful in applications for radiotherapy.

## Description

### FIELD OF THE INVENTION

The present invention relates to radiography and in particular to image storage assemblies that are useful for oncology or radiotherapy imaging and to a radiation image recording and reproducing method.

### BACKGROUND OF THE INVENTION

Conventional medical diagnostic imaging obviates to obtain an image of a patients internal anatomy, exposing the patient to a dose of X-rays, as low as possible. So fast imaging speeds are realised by mounting a duplitized or double-side coated silver halide radiographic element between a pair of fluorescent intensifying screens for imagewise exposure. Only a low percentage of the exposing X-radiation passing through the patient is directly absorbed by the silver halide emulsion layers, thereby forming a latent image within emulsion crystals of coated layers of said duplitized radiographic element. Most of the X-radiation that participates in image formation is absorbed by phosphor particles within the fluorescent screens and fluorescent light, promptly emitted by such intensifying screens becomes absorbed by the silver halide emulsion layers of the radiographic element. Examples of radiographic elements, constructions for medical diagnostic purposes are provided by EP-A's 0 890 873, 0 930 527, 1 045 282, 1, 103 849, 1 217 428 and by US-A's 4,425,425; 4,425,426; 4,414,310; 4,803,150; 4,900,652; 5,252,442; 5,989,799; and 6,403,276.

Radiation oncology is a field of radiology relating to the treatment of cancers, making use therefore of high energy X-radiation. This treatment is also known as "teletherapy", making use of powerful, high-energy X-radiation machines (often linear accelerators) or Co-60 units to exposure the cancerous tissues or tumors. The goal of such a treatment is to cure the patient by selectively killing the cancer while minimising damage to surrounding healthy tissues.

Such treatment is commonly carried out using high energy X-radiation, 4 to 25 MV. The X-radiation beams are very carefully mapped for intensity and energy. The patient is carefully imaged using a conventional diagnostic X-radiation unit, a CT scanner, and/or an MRI scanner to accurately locate the various tissues, healthy as well as cancerous, in the patient. Full knowledge of the treatment beam and the anatomy of the patient allows a dosimetrist to determine where and for how long the treatment X-radiation should be directed, and to predict the radiation dose to the patient.

Usually, this causes some healthy tissues to be overexposed. In order to reduce this effect, the dosimetrist specifies the shape of the beam that will be controlled by lead blockers at the source or "port" of the treatment device. This effectively acts as a substantially opaque block in front of parts of the patient's body, absorbing harmful X-radiation that would damage healthy tissues.

Three distinct types of imaging are carried out in radiation oncology. The first type of imaging is called "simulation". In this procedure, the patient is carefully imaged using a conventional diagnostic X-radiation unit, a conventional radiographic imaging film system, a storage or stimulable phosphor system, or a digital system. In addition, a CT scanner and/or MRI scanner may be used to accurately locate the patient's anatomy. These procedures are essentially like those used in diagnostic radiography. They are carried out using energies in the range from 50 to 150 kV with low doses of radiation. These images provide detailed information on the patient's anatomy, and the location of the cancer relative to other body parts. From the simulation images and/or CT/MRI data, a dosimetrist can determine where and for how long the treatment X-radiation should be directed. The dosimetrist makes use of a computer in order to predict the X-radiation dose for the patient. As this may lead to overexposure of some normal tissues, the dosimetrist will introduce one or more "blocks" or lead shields in order to block X-radiation from normal healthy anatomy. Alternatively, where available, the dosimetrist can shape the beam by specifying the positions for a multi-leaf collimator (MLC).

In order to determine and document that a treatment radiation beam is accurately aimed and is effectively killing the cancerous tissues, two other types of imaging are carried out during the course of the treatment. "Portal radiography" is generally the term used to describe such radiotherapy in the MV energy ranges, conducted through an opening or port in a radiation shield. The first type of portal imaging is known as "localisation" or "low dose portal" imaging in which the portal radiographic film is briefly exposed to the X-radiation passing through the patient with the lead shields removed and then with the lead shields in place. Exposure without the lead shields provides a faint image of anatomical features that can be used as orientation references near the targeted feature while the exposure with the lead shields superimposes a second image of the port area. This process insures that the lead shields are in the correct location relative to the patient's healthy tissues. Both exposures are made using a fraction of the total treatment dose, usually 1 to 4 monitor units out of a total dose of 45-150 monitor units, so that the patient receives less than 20 RAD's of radiation. If the patient and lead shields are accurately positioned relative to each other, the therapy treatment is carried out using a killing dose of X-radiation administered through the port. The patient typically receives from 50 to 300 RAD's, wherein 1 RAD corresponds with an energy absorption of 100 ergs per gram of tissue during treatment. The term "localization" thus refers to portal imaging that is used to locate the port in relation to the surrounding anatomy of the irradiated subject, wherein exposure times range from 1 to 10 seconds.

A second, less common form of "portal radiography" is known as "verification" or "high dose portal" imaging to verify the location of the cell-killing exposure. The purpose of this imaging is to record enough anatomical information to confirm that the cell-killing exposure was properly aligned with the targeted tissue. The imaging film/cassette assembly is kept in place behind the patient for the full duration of the treatment. The term "verification" thus refers to portal imaging that is used to record patient exposure through the port during radiotherapy. Typically exposure times range from 30 to 300 seconds. Verification films have only a single field, as the lead shields are in place, and are generally imaged at intervals during the treatment regime that may last for weeks. Portal radiographic imaging film, assembly and methods have been described, e.g., in US-A's 5,871,892 and 6,042,986; in which the same type of radiographic element can be used for both localization and portal imaging.

A radiographic phosphor panel contains a phosphor layer, wherein said phosphor is a crystalline material that responds to X-radiation on an imagewise basis. Radiographic phosphor panels can be classified, based on the type of phosphors, as prompt emission panels and image storage panels. Luminescent intensifying screens are the most common prompt emission panels and are generally used to generate visible light upon exposure to provide an image in radiographic silver halide materials. Storage phosphor panels comprise storage phosphors that have the capability of storing latent X-radiation images for later emission by stimulation with a laser beam in order to set free stored energy. Storage phosphors, also called photostimulable phosphors can be distinguished from the phosphors used in luminescent intensifying screens because the prompt emitting intensifying screen phosphors cannot store latent images for later emission. Rather, they immediately release or emit light upon irradiation. Various storage phosphors have been described, as e.g. in EP-A's 0 369 049, 0 399 662, 0 498 908, 0 751 200, 1 113 458, 1 137 015, 1 158 540, 1 316 969 and 1 316 970, as well as in US-A's 4,950,907; 5,066,864; 5,180,610; 5,289,512 and 5,874,744.

Storage phosphor systems for portal imaging as originally developed did not make use of a metal converter screen. However, this adversely affects image quality as pointed out in several publications as, e.g., by Wilenzink et al., Med. Phys., 14(3), 1987, pp. 389-392, and David et al., Med. Phys., 16(1), 1989, pp. 132-136. Subsequent teaching in this art e.g. suggests that 1 mm copper metal plate would enhance contrast and image quality, as exemplified e.g. by Weiser et al., Med. Phys. 17(1), 1990, pp. 122-125, and Roehrig et al., SPIE, 1231, 1990, pp. 492-497. Soon thereafter, aluminum, copper, tantalum, and lead metal plates were considered with storage phosphor screens as disclosed by Barnea et al., Med. Phys., 18(3), 1991, pp. 432-438. The conventional understanding in the art is that even storage phosphor panels require relatively thick metal screens to improve image quality. However, the weight of such image storage assemblies is considerable and creates a problem for users in the medical imaging community as light-weight cassettes are desired. Since the earliest teaching about the need for metal screens in image storage assemblies, the thickness of the metal screens has been set at 1 mm or more when copper is used and at 0.6 mm when lead is used. As set out in US-A 6,428,207 a thickness of about 0.1 to 0.75 mm for copper and from about 0.05 to about 0.4 mm for lead was preferred, and even more preferably, the thickness was from about 0.1 to about 0.6 mm for copper screens and from about 0.05 to about 0.3 mm for lead screens, although it was consistently believed until then that thick metal screens were required to avoid overexposure, especially for portal imaging. Heavy conventional image storage assemblies indeed provided desired high contrast images, but because of the thick metal screens used in order to provide the desired imaging features, they were very heavy and difficult and unsafe to carry throughout medical facilities. Medical users have tolerated this disadvantage as thick metal plates were believed to be necessary for desired imaging properties, although light-weight cassettes would provide a better processing.

### OBJECT AND SUMMARY OF THE INVENTION

Therefore it is an object to provide less heavy, more light-weight cassettes as a whole, without laying burden upon desired image properties as image contrast and image definition.

The above-mentioned advantageous effects have been realised by providing an X-ray imaging cassette having a cover side and a tube side, comprising inbetween a radiation image storage phosphor screen, plate or panel, from now on called "phosphor plate", and a metal filter sheet or foil, from now on called "filter foil", characterised in that said metal filter sheet is composed of tungsten and has a thickness in the range from 0.10 to 0.60 mm, and more preferably in the range from 0.10 to 0.30 mm.

A method for storing and reproducing a radiation image has also been claimed. Specific features for preferred embodiments of the invention are set out in the dependent claims.

Further advantages and embodiments of the present invention will become apparent from the following description.

### BRIEF DESCRIPTION OF THE DRAWINGS

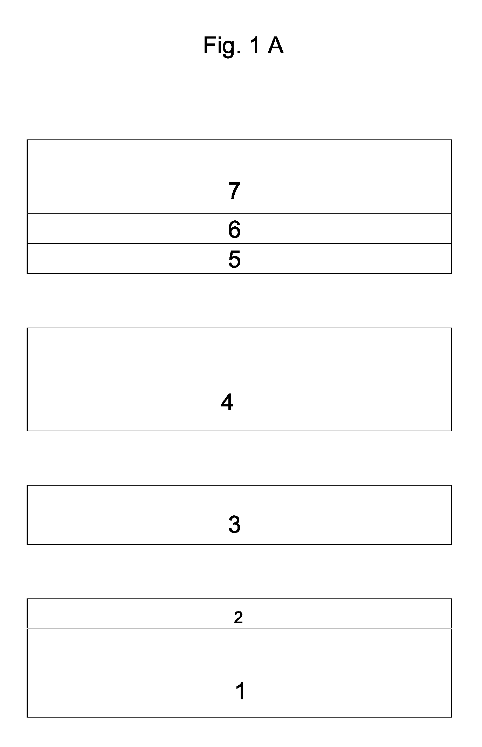

In Fig. 1A a layer arrangement for the radiotherapy cassette has schematically been given, wherein following consecutive parts are recognised (starting from (1) at the tube side of the X-ray imaging cassette, where radiation impinges upon the cassette), relative thicknesses of layers not being relevant or indicative for reality, as for each particular application another adapted layer thickness arrangement is, or may be, provided:
- cassette tube side (1);
- non-removable steel foil (2) (as magnetic counterpart for the magnetic sheet (5), foil (2) being non-removably attached to the cassette tube side (1);
- optionally removable (in order to make change of it possible for other specific applications) tungsten filter foil (3) having a preferred thickness between 0.10 and 0.30 mm in order to provide equilibrium at 6 MV, being in contact with the steel foil (2), and sandwiched between said steel foil (2) and storage phosphor plate (4);
- removable X-ray image storage phosphor plate (4) as central part between cassette tube side cover and opposite cassette cover;
- non-removably (but flexibly movable by means of (hastofaan) strips) attached magnetic sheet (5) acting as a means for magnetically closing the cassette between said magnetic sheet and steel foil non-removably attached to the cassette tube side, (said strips bridging the magnetic sheet (5) and the next layer in the direction of the cover side;
- non-removable lead (or lead compound) sheet (6), absorbing X-rays, having passed the X-ray image storage panel;
- cassette cover (7) in contact with the non-removable lead (or lead compound) sheet.

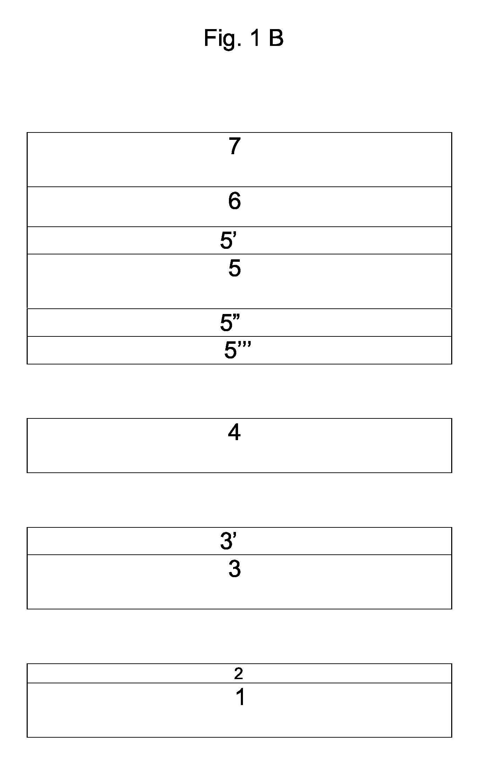

In Fig. 1B another layer arrangement for the radiotherapy cassette has schematically been given: wherein following changes have been applied versus in Fig. 1A:
- presence of a thin steel foil (5') besides the thicker magnetic foil (5) from Fig. 1A, upon a non-removable lead (or lead compound) sheet (6),
- presence of a fleece layer (5''') upon polymer layer (5");
- presence of fleece pads (3') upon tungsten filter foil (3).

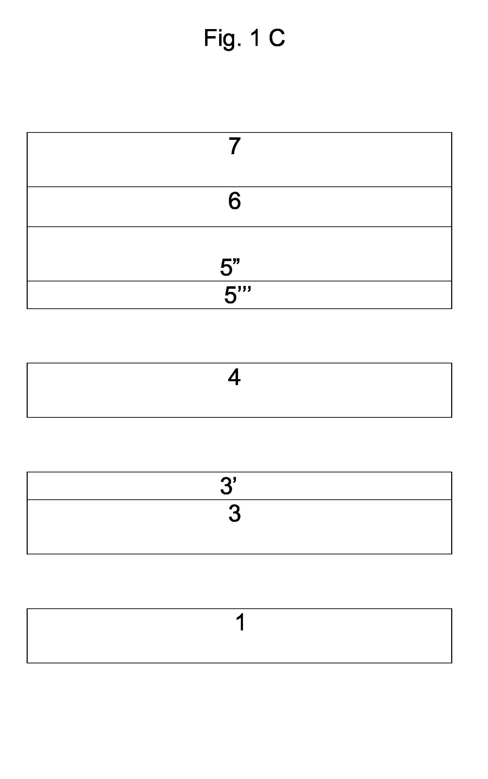

In Fig. 1C a further alternative layer arrangement for the radiotherapy cassette has schematically been given, wherein about a same layer arrangement has been given as in Fig. 1B, except for the absence of magnetic counterparts steel foil (2) and magnetic sheet (5) and thin steel foil (5'), wherein (5") stands for a tough polymer (e.g. polycarbonate) layer. The alternative closure system is not based upon magnetic forces but on mechanic pressure forces.

These arrangements are not limitative; other alternative arrangements will be described in the detailed description hereinafter.

### DETAILED DESCRIPTION OF THE INVENTION

According to the present invention an X-ray imaging cassette having a cover side and a tube side is thus provided, wherein said cassette comprises, inbetween cover and tube, a radiation image storage phosphor plate and a metal filter foil, in contact with each other, characterised in that said metal filter foil is composed of tungsten and has a thickness in the range from 0.10 to 0.60 mm, and more preferably in the range from 0.10 to 0.30 mm. In order to reach an equilibrium at 6 MV the tungsten filter foil in said X-ray imaging cassette has a particularly selected thickness of of 0.20 ± 0.05 mm.

As described hereinbefore, it is clear that X-rays are first reaching the tube side of the cassette assembly, and further pass through the tungsten filter foil, before reaching the storage phosphor panel and before further going out via the cover of said assembly. In favour of image quality, it is recommended to have a good contact between the tungsten filter foil and the storage phosphor plate: preferably said tungsten filter foil and storage phosphor plate are positioned adjacent to each other, which refers to the intimate contact between both the foil and the plate. Alternatively the tungsten foil and phosphor plate are separated by a gap in the range of 1 to 3 mm, which gap can be created by a compressible, preferably porous, layer adhesive to the filter foil or by air. According to the present invention, in the case wherein no direct contact between tungsten filter foil and storage phosphor plate is provided, in the X-ray cassette, a felt or felt-like sheet is present between said tube side and said storage phosphor plate. Said felt or felt-like sheet covers the filter over its whole surface or over only part of it, wherein under "only part of it" it is not excluded that only 1-5 % is covered in form of pads of felt or felt-like sheet. According to the present invention, in one particular embodiment, fleece pads made adhesive to the tungsten foil (on at least two differing sites thereof in order to facilitate removal of the storage phosphor plate after having been compressed before exposure between tungsten foil (4) and magnetic sheet (5) or polymer layer (5'), whether or not integrally covered with a fleece layer (5''') as a compressible porous material. A porous material provides as a particular advantage that air, enclosed between the storage phosphor plate and adjacent sheets or foil sat both sides, is easily removed before exposure. Direct contact of adjacent foils or layers at both sides of the storage phosphor plate provides accurate positioning and improved image quality indeed. After exposure however, the storage phosphor plate must be taken out of the cassette, without causing damaging of the storage phosphor plate and/or tungsten foil by streaks, stripes or scratches and therefor presence of such a porous material in form of a felt or felt-like sheet, such as a fleece layer at the cover side and as fleece pads adhered to the tungsten foil at the tube side is highly recommended.

A combination of features like reliability, durability, comfort and minimum weight in one cassette has further been realised by making use of an extremely light-weight tough plastic as NOVODUR®, which is 25 % lighter than metal cassettes, long lasting and shockproof. In order to provide a sufficiently good enclosure of all plates or panels, arranged between tube side and cover of the cassette of the present invention, it is highly recommended to provide a thicker bottom at the cassette cover at the back side, composed of the extremely light-weight tough plastic. Light-weight is further provided by not making use of the steel foil (2) and magnetic sheet (5) from Fig. 1A, that offer ability to magnetically close the cassette, but by replacing magnetic sheet (5) by a tough polymer sheet or layer, as shown in Fig. 1C. A very suitable polymer sheet or layer, offering good compression ability in favour of intimate contact of the storage phosphor plate with adjacent layers, is a polycarbonate polymer plate, without however being limitative to the composition of the polymer foil used. Such a polymer plate has a thickness in the range from about 1.2 mm to 1.8 mm, more preferably about 1.50 ± 0.25 mm and is in contact with a (preferably black) fleece layer integrally covering said polymer layer at the side of the storage phosphor plate, and in contact with a layer of lead or lead compounds, preventing backscattering while exposing the cassette to radiation, wherein said backscattering preventing layer is present at the cover side of the cassette, where non-absorbed radiation is leaving the cassette. At the side of the storage phosphor plate said (polycarbonate) polymer plate is preferably covered with a layer of BAYFOL®, from BAYER AG, Leverkusen, Germany, of 0.20 mm thickness, being black and electrically conducting and made adhesively sticking onto the (polycarbonate) polymer plate 'DIN 16801-Tfl-1,5-PC-glasklar' by means of a 'Transferkleber TT50'. The "backscattering preventing layer" mentioned above, having a thickness of about 0.15 mm, present at the cover side of the cassette, may be present between 'Transferkleber TT50' layers, wherein one at the side of the polycarbonate plate and foil is in contact with the BAYFOL® foil, whereas the other is adhered onto the lead foil in the direction of the cover plate.

The tungsten foil having the desired thickness of about 0.20 mm enables absorption of MV energy levels of X-radiation in the range of 4-50 MV, thereby releasing electrons and absorbing electrons that have been generated by said X-radiation before reaching the screen. Its preference above a 0.4 mm tantalum filter foil, is, besides its at least as good results with respect to image quality, related with its lighter weight, as envisaged. This however does not exclude use of a combination of tantalum and tungsten foils in contact with each other and having a total thickness inbetween 0.2 mm and 0.4 mm.

According to the present invention a foil of lead is further present, wherein said sheet of lead is arranged between cover and phosphor storage screen. Said foil of lead is in permanent contact with at least one of said storage phosphor plate or said cassette cover. Such a lead foil has a backscatter reducing function. Lead is known as a heavy metal, and it is clear that its thickness should be reduced up to a minimum level in the range from 0.05 up to 0.25 mm. A foil of aluminum or tungsten, whether or not present as such or glued to lead or lead compound layer forms an alternative embodiment. In an alternative form according to the present invention a lead compound is provided in a backscatter layer or foil as an oxide or a hydroxide of lead, which is dispersed in a binder and wherein said binder containing the said lead compound is a matrix of a polycondensation product of a metal alkoxide species. According to the present invention said X-ray cassette is provided with a sheet or foil of lead or lead compound, wherein said sheet or foil of lead or lead compound is further characterised in that it is protected with a coated layer selected from the group consisting of a lacquer layer, a polymeric (glue) layer and a felt cloth. This is a very interesting embodiment as contamination by lead should be avoided, the more when said lead (compound) foil is present in contact with the storage phosphor plate, and more particularly when such a foil is e.g. glued thereto.

In another embodiment an X-ray imaging cassette according to the present invention is provided with a lead or lead compound foil in permanent contact with the cover of the cassette, whereas the radiation image storage phosphor panel is removable and the tungsten filter screen is, or may optionally be, removable from the said cassette. Permanent contact is advantageously provided between phosphor plate and lead or lead compound foil e.g. by being glued thereto. In the case according to the present invention wherein an X-ray imaging cassette is provided with a non-removable backscatter sheet, said sheet is situated more close to the cover at the back side of the cassette than the radiation image storage phosphor plate.

In view of "low backscatter levels" causing a disturbing undesired image in a storage phosphor panel, a preferred support for the storage phosphor plate, if not self-supporting, is amorphous carbon(a-C), not only thanks to the black, radiation absorbing particles, but, to a more remarkable extent, thanks to the nature of that a-C material, generation very little backscatter. In a supported phosphor panel or screen used in the cassette according to the present invention, the thickness of such an amorphous carbon layer may range from 100 µm up to 3000 µm, a thickness between 500 µm and 2000 µm being preferred as a compromise between flexibility, strength, X-ray absorption and low backscatter. This may allow further presence of a thinner lead screen between the cover and the supported storage phosphor plate, wherein said lead screen further absorbs incident X-ray absorption. An X-ray imaging cassette according to the present invention is, in still another preferred embodiment, provided with a radiation image storage phosphor panel, wherein said radiation image storage phosphor panel is provided with a non-removable sheet or foil at the side more close to the cover than to the tube side, and wherein said non-removable sheet or foil is selected from the group consisting of lead, aluminum, amorphous carbon and an intensifying phosphor screen.

According to the present invention, apart from said non-removable backscatter layer, a lead or lead compound sheet in contact with the cover of said cassette is further separated from the radiation image storage panel by a non-removable magnetic sheet, making contact with the said radiation image storage phosphor panel, optionally separated therefrom by a fleece (as a compressible porous material) layer, integrally covering said magnetic sheet. This arrangement provides suitable handlability by magnetic closure of the cassette. According to a further preferred embodiment of the present invention the X-ray imaging cassette is magnetically closed as in the layer build-up illustrated in the Figures 1A and 1B, by the said non-removable magnetic sheet (5) in the cover part of the cassette (mentioned hereinbefore) and a non-removable steel foil (2) adjacent to the cassette tube side part. A steel foil (5'), having a non-critical thickness of about 50 µm, is, in another embodiment present between "magnetic sheet" and anti-backscatter, non-removable lead (or lead compound) sheet (6).

According to the present invention an X-ray imaging cassette is provided with a radiation image storage phosphor plate, which is a supported or non-supported storage phosphor layer, and which stores radiation having an energy in the range from 4 MV up to 50 MV and releases stored energy in form of light upon irradiation with light energy having a wavelength in the range of visible light or infrared radiation. During low dose portal imaging the patient is briefly exposed to energies in the range as set forth above over a region that is somewhat larger than the radiotherapy target area for the purpose of obtaining a discernible image of anatomy reference features outside the target area. This is in praxis immediately followed by a brief exposure through the port in the shields in order to create an image of the port superimposed on the broader region first exposure. Total exposure during low dose localisation imaging is limited to about 10 seconds or less. An image is envisaged in that way that confirms or guides alignment of the port for radiotherapy, further limiting exposure to the extent possible. The images in the location of the port are observed with respect to reference anatomy features, in order to more accurately align the port with the target area, before the radiotherapy exposure begins for a longer exposure time.

According to the present invention the X-ray imaging cassette has a supported or self-supporting storage phosphor plate or panel further comprising phosphor particles composed of elements having an atomic number 37 or more, as e.g. the preferred range of 39 or 57 through 71, representing the so-called "rare earth" elements or lanthanides.

As the storage phosphor panel is supported or self-supporting, it is clear that there is a difference in flexibility in both embodiments, depending on the rigidity of the arrangement. Flexible supports are generally polymeric in nature and include common polyesters, including polyesters, cellulose acetate, and polycarbonate films. Reflective supports, loaded with white pigments are e.g. titanium or barium sulfate and titanium dioxide (rutile or anatase type titanium dioxide) in favour of speed, without however being limeted thereto. Absorbing supports are e.g. polyester supports containing carbon black, in favour of image quality, without however being limeted thereto.

In one embodiment radiation image storage panels present in cassettes according to the present invention are provided by layers coated with divalent europium-doped bariumfluorohalide phosphors, wherein the halide-containing portion may be
(1) stoichiometrically equivalent with the fluorine portion as e.g. in the phosphor described in US-A 4,239,968,
(2) may be substoichiometrically present with respect to the fluorine portion as described e.g. in EP-A 0 021 342 or 0 345 904 and US-A 4,587,036, or
(3) may be superstoichiometrically present with respect to the fluorine portion as described e.g. in US-A 4,535,237.

BaFBr:Eu type phosphors further include europium activated barium-strontium-magnesium fluorobromide containing an effective amount of both strontium and magnesium as in EP-A 0 254 836; europium-doped barium fluorohalide photostimulable phosphor comprising an amount of oxygen sufficient to create a concentration of anion vacancies effective to substantially increase the stored photostimulable energy, compared to a non-oxygen-treated phosphor described in US-A's 5,227,254 and 5,380,599; and divalent europium activated barium fluorobromide containing as codopant samarium, and wherein the terminology barium fluorobromide stands for an empirical formula wherein (1) a minor part of the barium (less than 50 atom %) is replaced optionally by at least one metal selected from the group consisting of a monovalent alkali metal, a divalent alkaline earth metal other than barium, and a trivalent metal selected from the group consisting of Al, Ga, In, Tl, Sb, Bi, Y, and a rare earth metal selected from the group consisting of Ce, Pr, Nd, Gd, Tb, Dy, Ho, Er, Tm, Yb and Lu, (2) a minor part (less than 50 atom %) of the bromine is replaced by chlorine, and/or iodine, and (3) wherein fluorine is present stoichiometrically in a larger atom % than bromine taken alone or bromine combined with chlorine and/or iodine as in US-A 5,547,807; as well as the phosphors disclosed in the radiation image recording and reproducing methods described in EP-A's 0 111 892, 0 111 893. The phosphor set forth in US-A 4,239,968 is e.g. a phosphor selected from the group of alkaline earth metal fluorohalide phosphors and can be used for recording and reproducing a radiation image in the present invention, following the steps described there of
(i) causing a visible ray- or infrared ray-stimulable phosphor to absorb a radiation passing through an object, and
(ii) stimulating said phosphor with stimulation rays selected from visible rays and infrared rays to release the energy of the radiation stored therein as fluorescent light, characterized in that said phosphor is at least one phosphor selected from the group of alkaline earth metal fluorohalide phosphors. From the stimulation spectrum of said phosphors it can be learned that said kind of phosphor has high sensitivity to stimulation light of a He-Ne laser beam (633 nm) or, alternatively, to stimulation light of a laser diode of e.g. 658 nm (acting in the range from 650 nm to 666 nm), but poor photostimulability below 500 nm. The stimulated light (fluorescent light) is situated in the wavelength range of 350 to 450 nm with a peak at about 390 nm (ref. the periodical Radiology, September. 1983, p.834.). It can further be learned from said US-A 4,239,968 that it is desirable to use a visible ray (e.g. red light) stimulable phosphor rather than an infra-red ray-stimulable phosphor because the traps of an infra-red-stimulable phosphor are shallower than these of the visible ray-stimulable phosphor and, accordingly, the radiation image storage panel comprising the infra-red ray-stimulable phosphor exhibits a relatively rapid dark-decay (fading). For solving that problem it is desirable as explained in the same US-A 4,239,968 to use a photostimulable storage phosphor which has traps as deep as possible to avoid fading and to use for emptying said traps light rays having substantially higher photon energy (rays of short wavelength).

Attempts have been made to formulate phosphor compositions showing a stimulation spectrum in which the emission intensity at the stimulation wavelength of 500 nm is higher than the emission intensity at the stimulation wavelength of 600 nm. A suitable phosphor for said purpose, which is also suitable for use in the present invention has been described in US-A 4,535,238 in the form of a divalent europium activated barium fluorobromide phosphor having the bromine-containing portion stoichiometrically in excess of the fluorine. According to that US-A 4,535,238 the photostimulation of the phosphor can proceed effectively with light, even in the wavelength range of 400 to 550 nm.

Although BaFBr:Eu²⁺ storage phosphors, used in digital radiography, have a relatively high X-ray absorption in the range from 30-120 kV, which is a range relevant for general medical radiography, the absorption is lower than the X-ray absorption of most prompt-emitting phosphors used in screen/film radiography, like e.g. LaOBr:Tm, Gd₂O₂S:Tb and YTaO₄:Nb. Therefore, said screens comprising light-emitting luminescent phosphors will absorb a larger fraction of the irradiated X-ray quanta than BaFBr:Eu screens of equal thickness. The signal to noise ratio (SNR) of an X-ray image being proportional to the square-root of the absorbed X-ray dose, the images made with the said light-emitting screens will consequently be less noisy than images made with BaFBr:Eu screens having the same thickness. A larger fraction of X-ray quanta will be absorbed when thicker BaFBr:Eu screens are used. Use of thicker screens, however, leads to diffusion of light over larger distances in the screen, which causes deterioration of image resolution. For this reason, X-ray images made with digital radiography, using BaFBr screens, as disclosed in US-A 4,239,968, give a more noisy impression than images made with screen/film radiography. A more appropriate way to increase the X-ray absorption of phosphor plates is by increasing the intrinsic absorption of the phosphor. In BaFBr:Eu storage phosphors this can be achieved by partly substituting bromine by iodine. BaFX:Eu phosphors containing large amounts of iodine have been described e.g. in EP-A 0 142 734. Therefore, in a phosphor as disclosed in EP-A 0 142 734, the gain in image quality, due to the higher absorption of X-rays when more than 50% of iodine is included in the phosphor is offset by the lowering of the relative luminance.

Divalent europium activated barium fluorobromide phosphors suitable for use according to the present invention have further been described in EP-A 0 533 236 and in the corresponding US-A 5,422,220 and 5,547,807. In the said EP-A 0 533 236 a divalent europium activated stimulable phosphor is claimed wherein the stimulated light has a higher intensity when the stimulation proceeds with light of 550 nm, than when the stimulation proceeds with light of 600 nm. It is said that in said phosphor a "minor part" of bromine is replaced by chlorine and/or iodine. By minor part has to be understood less than 50 atom %.

Still other divalent europium activated barium fluorobromide phosphors suitable for use in screens or panels according to the present invention have been described in EP-A 0 533 234. In that EP-A 0 533 234 a process is described to prepare europium-doped alkaline earth metal fluorobromide phosphors, wherein fluorine is present in a larger atom % than bromine, and which have a stimulation spectrum that is clearly shifted to the shorter wavelength region. Therein use of shorter wavelength light in the photostimulation of phosphor panels containing phosphor particles dispersed in a binder is in favour of image-sharpness since the diffraction of stimulation light in the phosphor-binder layer containing dispersed phosphor particles acting as a kind of grating will decrease with decreasing wavelength. As is apparent from the examples in this EP-A 0 533 234 the ultimately obtained phosphor composition determines the optimum wavelength for its photostimulation and, therefore, the sensitivity of the phosphor in a specific scanning system containing a scanning light source emitting light in a narrow wavelength region.

Other preferred photostimulable phosphors according to the applications mentioned hereinbefore contain an alkaline earth metal selected from the group consisting of Sr, Mg and Ca with respect to barium in an atom percent in the range of 0.1 to 20 at %. From said alkaline earth metals Sr is most preferred for increasing the X-ray conversion efficiency of the phosphor. Therefore in a preferred embodiment strontium is recommended to be present in combination with barium and fluorine stoichiometrically in larger atom % than bromine alone or bromine combined with chlorine and/or iodine. Other preferred photostimulable phosphors mentioned in that application contain a rare earth metal selected from the group consisting of Ce, Pr, Nd, Gd, Tb, Dy, Ho, Er, Tm, Yb and Lu with respect to barium in an atom percent in the range of 10⁻³ to 10⁻¹ at %.
From said rare earth metals Gd is preferred for obtaining a shift of the maximum of the photostimulation spectrum of the phosphor to the shorter wavelengths.

The preferred phosphors of that application referred to hereinbefore are also advantageously used in the present invention the proviso that, as set forth hereinbefore, the wavelength region of the stimulating rays is between 500 and 700 nm.

Still other preferred photostimulable phosphors for use according to the present invention contain a trivalent metal selected from the group consisting of Al, Ga, In, Tl, Sb, Bi and Y with respect to barium in an atom percent in the range of 10⁻¹ to 10 at %. From said trivalent metals Bi is preferred for obtaining a shift of the maximum of the photostimulation spectrum of the phosphor to the shorter wavelengths.

Preferred phosphors for use according to this invention are further phosphors wherein fluorine is present stoichiometrically in a larger atom % than bromine taken alone or bromine combined with chlorine and/or iodine, e.g. fluorine is present in 3 to 12 atom % in excess over bromine or bromine combined with chlorine and/or iodine. Still other particularly suitable barium fluorobromide phosphors for use according to the present invention contain in addition to the main dopant Eu²⁺ at least Sm as codopant as described in EP-A 0 533 233 and in the corresponding US-A 5,629,125. Still other useful phosphors are those wherein Ba-ions are partially replaced by Ca-ions at the surface of the phosphors have been described in EP-A 0 736 586.

In digital radiography it can be advantageous to use photostimulable phosphors that can very effectively be stimulated by light with wavelength higher than 600 nm as for phosphors included for use in storage panels according to the present invention, since then the choice of small reliable lasers that can be used for stimulation (e.g. He-Ne, semi-conductor lasers, solid state lasers, etc) is very great so that the laser type does not dictate the dimensions of the apparatus for reading (stimulating) the stimulable phosphor plate.

More recently stimulable phosphors, giving a better signal-to-noise ratio, a higher speed, further being stimulable at wavelengths above 600 nm have therefore been described in US-A's 5,853,946 and 6,045,722. Therein a storage phosphor class has been described providing high X-ray absorption, combined with a high intensity of photostimulated emission, thus allowing to build a storage phosphor system for radiography yielding images that have at the same time a high sharpness and a low noise content, through a decreased level of X-ray quantum noise and a decreased level of fluorescence noise. Further said class of photostimulable phosphors provides a high X-ray absorption, combined with a high intensity of photostimulated emission, showing said high intensity of photostimulated emission when stimulated with light having a wavelength above 600 nm. Said photostimulable phosphors can further be used in panels for medical diagnosis, whereby the dose of X-ray administered to the patient can be lowered and the image quality of the diagnostic image enhanced: in a panel including said phosphor in dispersed form on photostimulation with light in the wavelength range above 600 nm images with very high signal-to-noise ratio are yielded.

A very useful and preferred method for the preparation of stimulable phosphors can be found in Research Disclosure Volume 358, February 1994 p 93 item 35841. In order to produce phosphors with a constant composition and, therefore, with a constant stimulation spectrum for use in storage phosphor panels, even in the presence of co-dopants that influence the position of the stimulation spectrum as e.g. samarium or an alkali metal, added to the raw mix of base materials in small amounts as prescribed in EP-A 0 533 234, a solution therefore has been proposed in US-A 5,517,034. Therein a method of recording and reproducing a penetrating radiation image has been proposed comprising the steps of:
(i) causing stimulable storage phosphors to absorb said penetrating radiation having passed through an object or emitted
   by an object and to store energy of said penetrating radiation,
(ii) stimulating said phosphors with stimulating light to release at least a part of said stored energy as fluorescent light and (iii) detecting said stimulation light, characterized in that said phosphors consist of a mixture of two or more individually prepared divalent europium doped bariumfluorohalide phosphors at least one of which contains (a) co-dopant(s) which co-determi-ne(s) the character of the stimulation spectrum of the co-doped phosphor.

Further particularly suitable divalent europium barium fluorobromide phosphors for use according to that invention correspond to the empirical formula (I) of EP-A 0 533 236 and contain in addition to the main dopant Eu²⁺ at least one alkali metal, preferably sodium or rubidium, as a co-dopant. Preferred photostimulable phosphors according to that application contain samarium with respect to barium in an atom percent in the range of 10⁻³ to 10 at %. Other preferred photostimulable phosphors according to that application contain an alkali metal selected from the group consisting of Li, Na, K, Rb and Cs, with respect to barium in an atom percent in the range of 10⁻² to 1 at %.

In praxis a maximum in the stimulation spectrum for e.g. lithium fluxed stimulable europium activated bariumfluorohalide phosphor can be found between 520 and 550 nm, whereas for cesium fluxed phosphor its maximum is situated between 570 and 630 nm. Maxima for the stimulation spectra of said phosphors after making a mixture thereof can be found at intermediate wavelengths. The stimulation spectrum of said mixture is further characterized in that the emission intensity at 500 nm stimulation is always lower than the emission intensity at 600 nm. The broadening of the obtained stimulation spectra is a further advantage resulting from the procedure of making blends in that the storage panel in which the stimulable phosphors are incorporated is sensitive to a broad region of stimulation wavelengths in the visible range of the wavelength spectrum. As a consequence the storage panel comprising a layer with the phosphor blends described hereinbefore may offer universal application possibilities from the point of view of stimulation with different stimulating light sources. Different stimulating light sources that may be applied are those that have been described in Research Dislosure No. 308117, December 1989.

Coverage of the phosphor or phosphors present as a sole phosphor or as a mixture of phosphors whether or not differing in chemical composition and present in one or more phosphor layer(s) in a screen is preferably in the range from about 50 g to 2500 g, more preferably from 200 g to 1750 g and still more preferably from 300 to 1500 g/m². Said one or more phosphor layers may have the same or a different layer thickness and/or a different weight ratio amount of pigment to binder and/or a different phosphor particle size or particle size distribution. It is general knowledge that sharper images with less noise are obtained with phosphor particles of smaller mean particle size, but light emission efficiency declines with decreasing particle size. Thus, the optimum mean particle size for a given application is a compromise between imaging speed and image sharpness desired. Preferred average grain sizes of the phosphor particles are in the range of 2 to 30 µm and more preferably in the range of 2 to 20 µm, in particular for BaFBr:Eu type phosphors.

In the phosphor layer(s), any phosphor or phosphor mixture may be coated depending on the objectives that have to be attained with the manufactured storage phosphor plates. Besides mixing fine grain phosphors with more coarse grain phosphors in order to increase the packing density, a gradient of crystal sizes may, if required, be build up in the storage panel. Principally this may be possible by coating only one phosphor layer, making use of gravitation forces, but with respect to reproducibility at least two different storage panels coated from phosphor layers comprising phosphors or phosphor mixtures in accordance with the present invention may be coated in the presence of a suitable binder, the layer nearest to the support consisting essentially of small phosphor particles or mixtures of different batches thereof with an average grain size of about 5 µm or less and thereover a mixed particle layer with an average grain size from 5 to 20 µm for the coarser phosphor particles, the smaller phosphor particles optionally being present as interstices of the larger phosphor particles dispersed in a suitable binder. Depending on the needs required the stimulable phosphors in accordance with the present invention or mixtures thereof may be arranged in a variable way in these coating constructions.

In one embodiment very suitable phosphors are phosphors according to the general formula (I) :

M¹⁺X.aM²⁺X'₂bM³⁺X''₃:cZ (I)

wherein:
M¹⁺ is at least one member selected from the group consisting of Li, Na, K, Cs and Rb,
M²⁺ is at least one member selected from the group consisting of Be, Mg, Ca, Sr, Ba, Zn, Cd, Cu, Pb and Ni,
M³⁺ is at least one member selected from the group consisting of Sc, Y, La, Ce, Pr, Nd, Pm, Sm, Eu, Gd, Tb, Dy, Ho, Er, Tm, Yb, Lu, Al, Bi, In and Ga,
Z is at least one member selected from the group Ga¹⁺, Ge²⁺, Sn²⁺, Sb³⁺ and As³⁺, X, X' and X" can be the same or different and each represents a halogen atom selected from the group consisting of F, Br, Cl, I and 0 ≤ a ≤ 1, 0 ≤ b ≤ 1 and 0 < c ≤ 0.2. Such phosphors have been disclosed in, e.g., US-A-5 736 069.

Highly preferred phosphors for use in a binderless phosphor plate as in storage phosphor layers for use in cassettes of this invention are CsX:Eu stimulable phosphors, wherein X represents a halide selected from the group consisting of Br, Cl and I. In a preferred embodiment according to the present invention the storage phosphor used in binderless phosphor plates is an alkali metal phosphor, and, more preferably a CsBr:Eu type phosphor.

Such phosphors are normally prepared by a method comprising the steps of :
- mixing said CsX with between 10⁻³ and 5 mol % of an Europium compound selected from the group consisting of EuX'₂, EuX'₃ and EuOX', X' being a member selected from the group consisting of F, Cl, Br and I,
- firing said mixture at a temperature above 450°C
- cooling said mixture and
- recovering the CsX:Eu phosphor.

Such needle-shaped phosphor are thus suitable for use in the storage phosphor layers used in the cassettes according to the present invention. A preferred example is a CsX:Eu stimulable phosphor, wherein X represents a halide selected from the group consisting of Br and Cl is used, prepared by a method comprising the steps of mixing said CsX with between 10⁻³ and 5 mol % of an Europium compound selected from the group consisting of EuX'₂, EuX'₃ and EuOX', X' being a member selected from the group consisting of F, Cl, Br and I; firing said mixture at a temperature above 450 °C cooling said mixture and recovering the CsX:Eu phosphor.

The method for preparing a binderless phosphor plate using these phosphors and a method for recording and reproducing an X-ray image using such screens can be used in the context of the present invention as described in WO01/3156 and in US-Application 01059004.

A factor determining the sensitivity of the screen is the thickness of the phosphor layer, being proportional to the amount of phosphor(s) coated. Said thickness may be within the range of from 1 to 1000 µm, preferably from 50 to 500 µm and more preferably from 100 to 300 µm. In case however that needle-shaped CsBr:Eu type phosphors are used, the phosphor layer may even be up to 1000 µm as has been set out in EP-A 1 113 458. Therein a binderless storage phosphor plate with needle shaped crystals is prepared, wherein the phosphor is an alkali halide phosphor and the needles show high [100] unit cell orientation in the plane of the screen in order to provide a stimulable phosphor plate useful in an X-ray recording system with a very good compromise between speed of the recording system (i.e. as low as possible patient dose) with an image with high sharpness and low noise.

An image storage phosphor plate used in cassettes according to the present invention can be prepared by the following manufacturing process, when no use is made of binderless phosphors. The phosphor layer can be applied to the support by any coating procedure, making use of solvents for the binder of the phosphor containing layer as well as of useful dispersing agents, useful plasticizers, useful fillers and subbing or interlayer layer compositions that have been described in extenso in the EP-A 0 510 753. Phosphor particles may be mixed with dissolved rubbery and/or elastomeric polymers, in a suitable mixing ratio in order to prepare a dispersion. Said dispersion is uniformly applied to a substrate by a known coating technique as e.g. doctor blade coating, roll coating, gravure coating or wire bar coating, and dried to form a storage phosphor layer. Further mechanical treatments like compression to lower the void ratio is not required within the scope of the present invention.

Useful dispersing agents to improve the dispersibility of the phosphor particles dispersed into the coating dispersion are described in EP-A 0 510 753 as well as a variety of additives that can be added to the phosphor layers such as a plasticizer for increasing the bonding between the binder and the phosphor particles in the phosphor layer and, according to the present invention, to a light-reflecting or absorbing filler and/or a colourant.

Useful plasticizers include phosphates such as triphenyl phosphate, tricresyl phosphate and diphenyl phosphate; phthalates such as diethyl phthalate and dimethoxyethyl phthalate; glycolates such as ethylphthalyl ethyl glycolate and butylphthalyl butyl glycolate; polymeric plastizers, e.g. and polyesters of polyethylene glycols with aliphatic dicarboxylic acids such as polyester of triethylene glycol with adipic acid and polyester of diethylene glycol with succinic acid. One or more binders providing structural coherence to the layers may be useful and are those conventionally used for this purpose in the art. They can be chosen from a wide variety of known organic polymers that are transparent to X-radiation, stimulating and emitted radiation. Binder materials commonly used for this purpose include but are not limited to, natural polymers such as proteins (for example gelatins), polysaccharides (such as dextrans), poly(vinyl acetate), ethyl cellulose, vinylidene chloride polymers, cellulose acetate butyrate, polyvinyl alcohol, sodium o-sulfobenzaldehyde acetal of poly(vinyl alcohol), chlorosulfonated poly(ethylene), a mixture of macromolecular bisphenol poly(carbonates), and copolymers comprising bisphenol carbonates and poly(alkylene oxides), aqueous ethanol soluble nylons, poly(alkyl acrylates and methacrylates) and copolymers of poly(alkyl acrylates and methacrylates and acrylic acid or methacrylic acid) and poly(vinyl butryal) and poly(urethanes) elastomers. Mixtures of binders can be used if desired.

The stimulable phosphor is preferably protected against the influence of moisture by adhering thereto chemically or physically a hydrophobic or hydrophobizing substance. Suitable substances for said purpose are described e.g. in US-A 4,138,361.

In the composition of a storage panel, one or more additional layers are occasionally provided between the support and the phosphor containing layer, having subbing or interlayer layer compositions, in order to improve the bonding between the support and the phosphor layer, or in order to improve the sensitivity of the screen or the sharpness and resolution of an image provided thereby. For instance, a subbing layer or an adhesive layer may be provided by coating polymer material over the surface of the support on the phosphor layer side.

Additional layer(s) may be coated on the support either as a backing layer or interposed between the support and the intermediate layer, the said intermediate layer and the phosphor containing layer(s). Several of said additional layers may be applied in combination.

In the preparation of the phosphor plate having a primer layer between the substrate and the layer containing the phosphor(s), the primer layer is provided on the substrate beforehand, and then the phosphor dispersion is applied to the primer layer and dried to form the fluorescent layer.

When the phosphors are used in combination with a binder to prepare a screen or a panel according to the present invention, the phosphor particles are intimately dispersed in a solution of the binder and then coated on the support and dried. The coating of the present phosphor binder layer may proceed according to any usual technique, e.g. by spraying, dip-coating or doctor blade coating. After coating, the solvent(s) of the coating mixture is (are) removed by evaporation, e.g. by drying in a hot (60°C) air current.

An ultrasonic treatment can be applied to improve the packing density and to perform the de-aeration of the phosphor-binder combination. Before the optional application of a protective coating the phosphor-binder layer may be calendered to improve the packing density (i.e. the number of grams of phosphor per cm³ of dry coating). After applying the coating dispersion onto the support, the coating dispersion is heated slowly to dryness in order to complete the formation of a phosphor layer. In order to remove as much as possible entrapped air in the phosphor coating composition it can be subjected to an ultra-sonic treatment before coating.

After the formation of the phosphor layer, a protective layer is generally provided on top of the fluorescent layer.

Correlating features of roughness and thickness of the protective coating conferring to the screens or panels of the present invention having desirable and unexpected properties of ease of manipulation and excellent image sharpness have been described in the EP-A's 0 510 754 and 1 318 525.

The protective coating may advantageously be provided by means of screen printing (silk-screen printing) or be applied by a rotary screen printing device as has been described in detail in the said EP-A 0 510 753.

Very useful radiation curable compositions for forming a protective coating contain as primary components:
(1) a crosslinkable prepolymer or oligomer, or even combined with a polymer that is soluble in the reactive diluent monomer.
(2) a reactive diluent monomer, and in the case of an UV curable formulation
(3) a photoinitiator.

Examples of suitable prepolymers for use in a radiation-curable composition applied to the storage panel according to the present invention are the following: unsaturated polyesters, e.g. polyester acrylates; urethane modified unsaturated polyesters, e.g. urethane-polyester acrylates. Liquid polyesters having an acrylic group as a terminal group, e.g. saturated copolyesters which have been provided with acryltype end groups are described in published EP-A 207 257 and Radiat. Phys. Chem., Vol. 33, No. 5, 443-450 (1989). The latter liquid copolyesters are substantially free from low molecular weight, unsaturated monomers and other volatile substances and are of very low toxicity (ref. the journal Adhasion 1990 Heft 12, page 12). The preparation of a large variety of radiation-curable acrylic polyesters is given in German Offenlegungsschrift No. 2838691. Mixtures of two or more of said prepolymers may be used. A survey of UV-curable coating compositions is given e.g. in the journal "Coating" 9/88, p. 348-353. The protective overcoat layer may contain a variety of agents designed to enhance its utility as e.g. by agents including solid particulate materials or by matting agents as described in GB-A 1,534,154 and antistatic agents as described in WO 96/11481.

When the radiation-curing is carried out with ultraviolet radiation (UV), a photoinitiator is present in the coating composition to serve as a catalyst to initiate the polymerization of the monomers and their optional cross-linking with the pre-polymers resulting in curing of the coated protective layer composition. A photosensitizer for accelerating the effect of the photoinitiator may be present. Photoinitiators suitable for use in UV-curable coating compositions belong to the class of organic carbonyl compounds, for example, benzoin ether series compounds such as benzoin isopropyl, isobutylether; benzil ketal series compounds; ketoxime esters; benzophenone series compounds such as benzophenone, o-benzoylmethylbenzoate; acetophenone series compounds such as acetophenone, trichloroacetophenone, 1,1-dichloroacetophenone, 2,2-diethoxyacetophenone, 2,2-dimethoxy-2-phenylacetophenone; thioxanthone series compounds such as 2-chlorothioxanthone, 2-ethylthioxanthone; and compounds such as 2-hydroxy-2-methylpropiophenone, 2-hydroxy-4'-isopropyl-2-methylpropiophenone, 1-hydroxycyclohexylphenylketone, etc..

A particularly preferred photoinitiator is 2-hydroxy-2methyl-1-phenyl-propan-1-one which product is marketed by E. Merck, Darmstadt, Germany, under the tradename DAROCUR 1173. The above mentioned photopolymerisation initiators may be used alone or as a mixture of two or more. Examples of suitable photosensitisers are particular aromatic amino compounds as described e.g. in GB-A 1,314,556, 1,486,911, US-A 4,255,513 and merocyanine and carbostyril compounds as described in US-A 4,282,309.

When using ultraviolet radiation as curing source the photoinitiator which should be added to the coating solution will to a more or less extent also absorb the light emitted by the phosphor thereby impairing the sensitivity of the radiographic screen, particularly when a phosphor emitting UV or blue light is used. Electron beam curing may therefore be more effective. The protective coating of the present storage panel is given an embossed structure following the coating stage by passing the uncured or slightly cured coating through the nip of pressure rol-lers wherein the roller contacting said coating has a micro-relief structure, e.g. giving the coating an embossed structure so as to obtain relief parts as has been described e.g. in EP-A's 455 309 and 456 318.

A mixture of storage phosphors can be used, and particularly a mixture of storage phosphors containing iodide is useful. If more than one storage phosphor layer is used, those layers can be composed of the same or different storage phosphors and the same or different binders. The multiple phosphor layers can also have the same or different thickness. The amount of the one or more storage phosphors in the phosphor layers is generally at least 50 wt%, and preferably from about 80 up to even 98 wt%, based on total dry layer weight.

Any conventional ratio of storage phosphor to binder, if present, can be used in the storage phosphor layer present in the cassette assembly of the present invention. Generally thinner storage phosphor layers and sharper images are obtained when a high weight ratio of storage phosphor to binder is used. Preferably storage phosphor to binder weight ratios are in the range of from about 7:1 to about 30:1. More or less binder can be used if desired for specific applications. The one or more storage phosphor layers may include other addenda that are commonly employed for various purposes, including but not limited to reducing agents (such as oxysulfur reducing agents), phosphites and organotin compounds to prevent yellowing, dyes and pigments for light absorption, plasticizers, dispersing aids, surfactants, and antistatic agents, all in conventional amounts. The one or more storage phosphor layers generally have a total dry thickness of at least 50 µm, and preferably from about 100 µm to about 400 µm. The phosphor storage screens of this invention preferably include a protective overcoat layer disposed on the outer storage phosphor layer. This layer is substantially clear and transparent to the light emitted by the storage phosphor and provides abrasion and scratch resistance and durability. It may also be desirable for the overcoat layer to provide a barrier to water or water vapor that may degrade the performance of the storage phosphor. Further, it may be desirable to incorporate components into the overcoat layer that prevent yellowing of the phosphor storage screen.

The protective overcoat layer can extend over the phosphor storage screen to seal the edges of the phosphor layer(s) or a separate seal may be applied using the same composition as that of the overcoat or a composition differing therefrom. With respect to protection against moisture presence of a protective parylene layer as described e.g. in EP-A's 1 286 362, 1 286 364 and 1 286 365 is highly recommended.

The tungsten filter foil of reduced thickness suitable for use in the cassette according to the present invention can take any convenient conventional form. While metal filter foils are most easily fabricated as thin foils, they are often mounted on radiation transparent backings to facilitate handling. Convenient metals for foil fabrication are in the atomic number range of from 22 (titanium) to 82 (lead). Metals such as copper, lead, tungsten and iron have been most commonly used for metal filter foil fabrication. The tungsten filter foils used in the practice of this invention are thinner than conventional screens. They typically range from about 0.10 to 0.30 mm in thickness, with a most preferred thickness of about 0.20 mm. This most preferred thickness more particularly relates to attaining equilibrium at an exposure of 6 MV.

Instead of employing separate metal foils and storage phosphor plates, it is possible to integrate both functions into a single element by coating a storage phosphor layer onto a thin tungsten filter foil. Those thin tungsten filter foils would have a desired thickness within the ranges described above.

Before use the storage phosphor plates are in an advantageous embodiment, seperately packed in packages as described in EP-A 1 387 365, more particularly as such packages are offering a adequate protection against oxygen and moisture. The X-ray cassettes according to the present invention are generally used by exposing the storage phosphor plate to X-radiation that has passed through a patient being imaged, wherein the said X-ray radiation passes through the thin tungsten filter foil before the storage phosphor layer is passed, and is stored imagewise in the storage phosphor layer or layers. The storage phosphor plate is then scanned with suitable stimulating electromagnetic radiation (such as visible light or infrared radiation) to sequentially release the stored radiation as a light emission. The stimulating radiation is directed to the storage phosphor layer before it passes through the metal screen. The emitted light is then electronically converted to an image and either printed, stored, or transmitted elsewhere.

According to the present invention a method for storing and reproducing a radiation image comprises the steps of:
- (tightly, but not limited thereto) mounting a radiation image storage panel in an X-ray imaging cassette according to the present invention;
- exposing to irradiation the said cassette by means of a radiation source having an energy in the range from 1 kV up to 50 MV, and, more preferably for particular applications related with radiotherapy, in the range from 4 MV up to 50 MV, wherein said the object to be examined is situated between radiation source and cassette and wherein radiation is impinging first onto the tube side of the said cassette;
- capturing said radiation by the radiation image storage panel of radiation having penetrated through an object, a radiation having been emitted by an object, or a radiation having been scattered or diffracted by an object in order to store energy of the applied radiation in form of a latent image on the image storage layer of the storage panel;
- discharging the cassette by taking out the storage phosphor panel;
- irradiating the image storage panel on the side of image storage layer with stimulating light in the visible or infrared range of the wavelength spectrum in order to excite the phosphor in the storage phosphor layer so that the energy stored in the storage layer in the form of a latent image is released in form of light;
- collecting the light released from the storage phosphor layer by light-collecting means;
- converting the collected light into a series of electric signals; and
- producing an image corresponding to the latent image from the electric signals.

According to the present invention use of an X-ray imaging cassette is provided in applications for radiotherapy, more preferably for low dose and high dose portal imaging.

The phosphor plate as such is very suitable for use in dosimetric applications, related with radiotherapy, e.g. when it is impossible to put the cassette as such in a gap that is too small. In that case the phosphor plate should be protected and should preferably be put in an envelope.

It is further clear that a cassette according to the present invention may be used in applications wherein lower exposure energies are used, e.g. in the kV ranges as for mammography and chest imaging.

The following Examples are illustrative for preferred embodiments of the present invention, without however limiting them thereto.

### EXAMPLES

A phosphor plate was prepared by coating of a BaSrFBr:Eu storage phosphor lacquer on a polyethylene terephthalate support having reflecting or absorbing properties and providing it with an EB-cured protective coating. Said phosphor plate was inserted in a cassette, having a layer arrangement as in the Figure 1C.

The phosphor was stimulated with an image scanner made up with a laser diode having a stimulation wavelength of 658 nm (range between 650 nm and 666 nm). The beam of a 50 mW laser diode was focussed to a small spot of 60 µm (FWMH) (60 ± 10 µm) with an optic containing a beam expander and a collimating lens. A polygon mirror having a hexagonal configuration was used to scan the small laserspot over the entire width of a phosphor sample. During this scanning procedure the phosphor was stimulated and the emission light was captured by an array of optical fibers which were sited on one line. At the other end of the optical fibers, being mounted in a circle, a photomultiplier was installed.

In order to attenuate the stimulating light an optical filter, type BG3 from SCHOTT, was placed between the fiber and the photomultiplier. In this way only the light emitted by the phosphor was measured. The small current of the photomultiplier was first amplified with an I/V convertor and digitalised with an A/D convertor.

The measuring set up was connected with a HP 9826 computer and a HP 6944 multiprogrammer to controll the measurement. Starting the procedure an electronic shutter was closed to shut down the laser.

A phosphor sample measuring 15 cm x 15 cm was used for examination of glandular tissue (prostate).

The X-ray cassette in NOVODUR® plastic material was consecutively provided, between tube side and cover side, with a steel foil, fleece pads (whether or not present in the different experiments explained hereinafter), a metal screen (varying in thickness and composition), a supported storage phosphor layer, a fleece or felt cloth, a magnetic counterfoil or a cardboard plate, a polycarbonate plate and a lead backscatter screen (having a thickness of 0.15 µm).

The radiation dose was measured with a FARMER dosemeter. Between the X-ray source and the phosphor layer a "QC PHANTOM QC-3 SERIAL-143" (manufactured by CCMB for Masthead Imaging Corporation, Canada) having as calibrated and corrected frequencies 0.75, 0.43, 0.245, 0.20 and 0.10 line pairs per mm. After exposure the sample was put into the laser scanner. To read out one line the shutter was opened and the galvanometer was moved linearly. During the scanning procedure the emitted light was measured continuously with the A/D convertor at a sampling rate frequency of 100 kHz and stored within a memory card in the multiprogrammer. One scan thus contained 100000 pixels. Once the scan was complete the shutter was closed again and the galvano-meter was put on his original position again.

The data of the scan line were transferred from the memory card in the multiprogrammer to the computer where said data were analysed. A first correction took into account the sensitivity variation of the scan line with the distance.

Therefore a calibration scan was measured previously for a phosphor sample that was exposed quite homogeneously. A second correction took into account the amount of X-ray dose by dividing said values by the said dose amount.

The following Examples are illustrative for preferred embodiments of the present invention, without however being limitative.

### Experiment 1 (comparative - copper filter foil)

An assembly with a copper filter foil having a thickness of 1.5 mm was examined with and without felt cloth inbetween copper foil and storage phosphor plate. 6 and 18 MV exposures provides better images (less haze) without presence of a felt cloth. A thinner copper foil provides qualitatively better images in case of 6 MV exposure.

### Experiment 2 (comparative - tungsten and tantalum filters, thickness variation)

An assembly with a tungsten filter foil having a thickness of 0.76 mm was examined without felt cloth inbetween tungsten filter and storage phosphor plate. A 6 MV exposure provided a much better better result than obtained for a copper filter foil having the same thickness. Moreover if compared with tantalum filter foils having a thickness of 1.0 mm and 0.8 mm respectively, the tungsten filter foil showed about the same results (contrast, image quality) if compared with the 0.8 mm tantalum filter plate, and a better result than the 1.0 mm tantalum filter foil. At the other hand for exposures with an energy of 4, 6 and 18 MV respectively, it was shown that the tungsten filter foil having a thickness of 0.76 mm provided a lower image quality after exposure of the QC-3 PHANTOM, if compared with cassettes having tantalum filter foils with a thickness of 0.2 mm, 0.4 mm and 0.6 mm respectively.

### Experiment 3 (inventive - tungsten filter, thickness variation)

Selecting the best filter foil thickness compromise in common MV-range exposures for portal imaging with a technical phantom, when making use of an assembly of a storage phosphor plate with a tungsten filter foil having thicknesses of 0.2 mm, 0.4 mm and 0.6 mm respectively, clearly shows the most excellent results with respect to image quality for the 0.2 mm thick tungsten filter foil.

Moreover its significant light-weight advantage could not be overlooked and was highly appreciated in the practical evaluation.

### Experiment 4 (comparative - tungsten filter foil, thickness variation)

Just as in experiment 3 assemblies with tungsten filter foils having the same different thicknesses were examined, but with a cardboard inbetween storage phosphor plate and cover side of the cassette now, thus providing very good direct contact by pressure of the storage phosphor plate against the tungsten filter foil. It was established that presence of tungsten filter foils provided most excellent results with respect to image quality for the 0.2 mm thick tungsten filter foil again, if compared with tungsten filter foils having a thickness of 0.4 mm and 0.6 mm respectively, and even better than in the former Experiment 3 without cardboard. At 6 and 18 MV exposure excellent images were obtained and it was moreover clear that presence of a thin lead foil as a backscatter layer, preferably in direct contact with the storage phosphor panel, provided a still better image quality.

### Experiment 5 (inventive tungsten filter foil, comparison with tantalum filter foil)

In a further experiment an assembly of the BaFBr:Eu-type storage phosphor plate in close contact with a tungsten filter foil having a thickness of 0.2 mm was compared with a tantalum filter having a thickness of 0.4 mm and 0.6 mm respectively.

All of the three assemblies were compared with an assembly without a metal filter.

Technical phantom exposures with a cobalt source as well as with a linear accelerator (6 and 18 MV) proved that image quality was clearly worse when no metal filter was used during exposure.

The 0.2 mm tungsten filter and the 0.4 mm tantalum filter foils provided a comparable, excellent image quality, whereas the 0.6 mm thick tantalum filter foil did not improve image quality and even showed a little lower contrast-to-noise ratio.

The above-mentioned tungsten filter foil thickness of 0.2 mm was selected as providing the best results and was preferred above the tantalum filter foil having a thickness of 0.4 mm, thanks to the weight reduction of the whole cassette assembly.

### Experiment 6

Different systems with respect to the contact between metal filter and storage phosphor plate were tested in technical QC-3 PHANTOM exposures at the cobalt source and Linac (6 and 18 MV Linac exposures). An assembly with a tungsten filter foil having a thickness of 0.2 mm was used for this experiment, whereas optimal contact between tungsten filter foil and phosphor plate was established using a magnetic cassette 'pressure' system inside the cassette. This cassette was compared with a cassette in which an extra polycarbonate plate was provided in the cassette cover to enhance the contact between tantalum filter an phosphor plate. In a third assembly a polyester layer was laminated onto the tungsten filter foil, separating the tungsten filter foil from the phosphor plate by at least 43 µm.

No relevant differences in image quality have been measured for the cassette with "magnetic pressure system" and the one with "extra layers in the cassette cover".

The presence of an extra layer inbetween tantalum filter foil and storage phosphor plate was clearly resulting in a lower image resolution.

### Experiment 7

Assemblies with a Genrad® storage phosphor plate and a tantalum filter foil having a thickness of 0.4 mm and a tungsten filter foil of 0.2 mm respectively, were examined while double contrast exposing the assembly to 6 and 18 MV. It was shown that this double contrast low dose 'localisation' imaging produced good image quality for both foils, but with a preference for the tungsten filter foil, thanks to its lighter weight.

Having described in detail preferred embodiments of the current invention, it will now be apparent to those skilled in the art that numerous modifications can be made therein without departing from the scope of the invention as defined in the appending claims.

## Claims

1. An X-ray imaging cassette having a cover side and a tube side, comprising inbetween a radiation image storage phosphor plate or panel and a metal filter foil, **characterised in that** said metal filter foil is composed of tungsten and has a thickness in the range from 0.10 to 0.60 mm.

2. An X-ray imaging cassette according to claim 1, wherein said thickness is in the range from 0.10 to 0.30 mm.

3. An X-ray imaging cassette according to claim 1 or 2, wherein said thickness is 0.20 ± 0.05 mm.

4. An X-ray cassette according to any one of the claims 1 to 3, wherein a compressible porous material, present between said tube side and said storage phosphor plate, is in direct contact with said screen or panel.

5. An X-ray cassette according to any of the claims 1 to 4, wherein a sheet or foil of lead is present, wherein said sheet of lead is arranged between cover side and phosphor storage screen.

6. An X-ray cassette according to claim 5, wherein said sheet or foil of lead is an oxide or a hydroxide of lead, which is dispersed in a binder and wherein said binder containing the lead compound is a matrix of a polycondensation product of a metal alkoxide species.

7. An X-ray cassette according to claim 5 or 6, wherein said sheet or foil of lead or lead compound is further **characterised in that** it is protected with a coated layer selected from the group consisting of a lacquer layer, a polymeric layer, a fleece layer and a felt cloth.

8. An X-ray imaging cassette according to any one of the claims 5 to 7, wherein said lead or lead compound foil is in permanent contact with the cover of the cassette, whereas the radiation image storage phosphor plate is removable and the tungsten filter foil is optionnally removable from the said cassette.

9. An X-ray imaging cassette according to any one of the claims 5 to 8, wherein further a non-removable backscatter sheet is situated more close to the cover of the cassette than to the radiation image storage phosphor plate.

10. An X-ray imaging cassette according to claim 9, wherein said non-removable sheet or foil is selected from the group consisting of lead, aluminum, amorphous carbon and an intensifying phosphor plate.

11. An X-ray imaging cassette according to any one of the claims 4 to 10, wherein a lead or lead compound sheet in contact with the cover of said cassette is separated from the radiation image storage panel by a non-removable magnetic sheet, making contact with the said radiation image storage phosphor plate.

12. An X-ray imaging cassette according to claim 11, wherein the said cassette is magnetically closed by the said non-removable magnetic sheet in the cover part of the cassette and a non-removable steel foil adjacent to the tube side of the cassette.

13. An X-ray cassette according to any one of the claims 1 to 3, wherein a tough polymer plate covered with a compressible porous material is present between said cover side and said storage phosphor plate.

14. An X-ray cassette according to claim 13, wherein a magnetic sheet in the cover part of the cassette and a steel foil adjacent to the tube side of the cassette is absent in the layer arrangement of the cassette.

15. An X-ray imaging cassette according to any one of the claims 1 to 14, wherein said radiation image storage phosphor plate is a supported or non-supported storage phosphor layer which stores radiation having an energy in the range from 4 MV up to 50 MV and releases stored energy in form of light upon irradiation with light energy having a wavelength in the range of visible light or infrared radiation.

16. An X-ray imaging cassette according to any one of the claims 1 to 15, wherein said storage plate or panel further comprises phosphor particles having a composition wherein an atomic element is present having an atomic number 37 or more.

17. A method for storing and reproducing a radiation image which comprises the steps of:
- mounting a radiation image storage panel in an X-ray imaging cassette according to any one of the claims 1 to 16;
- exposing to irradiation the said cassette by means of a radiation source having an energy in the range from 1 kV up to 50 MV, wherein said the object to be examined is situated between radiation source and cassette and wherein radiation is impinging first onto the tube side of the said cassette;
- capturing said radiation by the radiation image storage panel of radiation having penetrated through an object, a radiation having been emitted by an object, or a radiation having been scattered or diffracted by an object in order to store energy of the applied radiation in form of a latent image on the image storage layer of the storage panel;
- discharging the cassette by taking out the storage phosphor panel;
- irradiating the image storage panel on the side of image storage layer with stimulating light in the visible or infrared range of the wavelength spectrum in order to excite the phosphor in the storage phosphor layer so that the energy stored in the storage layer in the form of a latent image is released in form of light;
- collecting the light released from the storage phosphor layer by light-collecting means;
- converting the collected light into a series of electric signals; and
- producing an image corresponding to the latent image from the electric signals.

18. Method according to claim 17, wherein the step of exposing to irradiation the said cassette proceeds by means of a radiation source having an energy in the range from 4 MV up to 50 MV.

19. Use of an X-ray imaging cassette according to any one of the claims 1 to 16 in applications for radiotherapy.
